# EUROPEAN PATENT APPLICATION

(11) **EP 4 821 504 A1**
(43) Date of publication of application: **14.10.2026**
(21) Application number: 25825160.2
(22) Date of filing: 24.06.2025
(51) Int. Cl.: A61K 38/12, A61K 9/20, A61K 47/20, A61K 47/38, A61P 35/00

(54) **SOLID DISPERSION AND COMPOSITION CONTAINING SAME**

(30) Priority: 25.06.2024 JP 2024102246
(71) Applicant: CHUGAI SEIYAKU KABUSHIKI KAISHA, Tokyo 115-8543 (JP)
(72) Inventor: KIMURA Ryosuke, Tokyo 115-8543 (JP); MINAMISAKAMOTO Mana, Yokohama City, Kanagawa 244-8602 (JP); NAKAMURA Kazuya, Yokohama City, Kanagawa 244-8602 (JP); NAKAE Shinichi, Tokyo 115-8543 (JP)
(74) Representative: Vossius & Partner Patentanwälte Rechtsanwälte mbB
(86) International application number: PCT/JP2025/022623
(87) International publication number: WO 2026/004834

(57) **Abstract**

A solid dispersion comprising a component (1) that is one or more selected from the group consisting of a compound represented by formula (I), pharmaceutically acceptable salts thereof, and pharmaceutically acceptable solvates thereof, and a component (2) that is one or more polymers selected from the group consisting of hydroxypropyl methylcellulose and derivatives thereof, wherein a total content of the component (2) per 1 part by mass of a total content of the component (1) is more than 0.1 parts by mass and less than 1.0 part by mass. A composition comprising the solid dispersion and an alkyl sulfate.

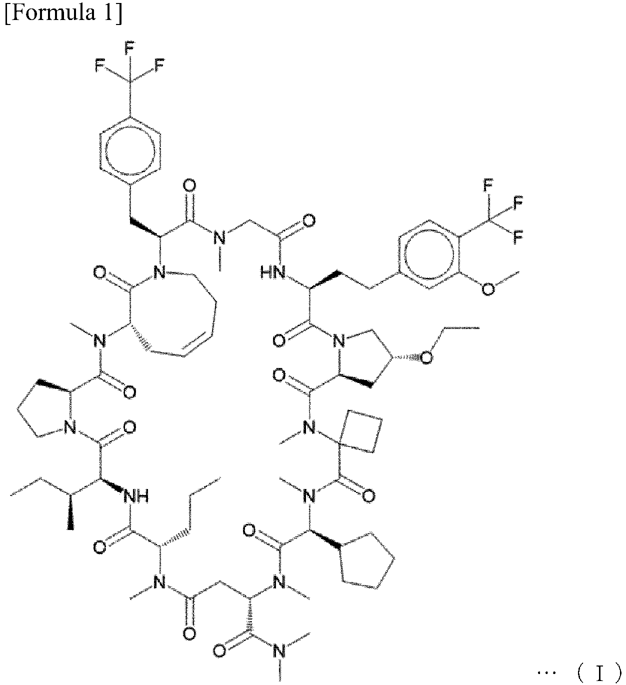

## Description

### Technical Field

The present invention relates to a solid dispersion and a composition containing the same. More specifically, the present invention relates to a solid dispersion containing a cyclic peptide compound and hydroxypropyl methylcellulose or a derivative thereof, and to a composition containing the solid dispersion and an alkyl sulfate.

### Background Art

RAS is a protein belonging to the small GTPase family, of which KRAS, NRAS, and HRAS are known. RAS is defined as activated or inactivated depending on its binding state to GDP or GTP, and it is activated by the exchange reaction of GDP to GTP by GEF (guanine nucleotide exchange factor) and inactivated by the hydrolysis reaction of GTP by GAP (GTPase-activating proteins) (Non Patent Literature 1). Activated RAS induces cell proliferation, survival, and differentiation by activating various downstream signals such as the MAPK pathway, PI3K/Akt pathway, and RAL pathway, and constitutive activation of RAS plays an important role in the development and progression of cancer. In cancer, the RAS-RAF-MEK-ERK pathway is known to be activated due to the activation of RAS upstream signaling, constitutive activation of RAS, and/or activating mutations of RAS (Non Patent Literature 2). These activating mutations of RAS have been found in numerous types of cancer. G12, G13, and Q61 are known as RAS mutation hotspots, and mutations are frequently found at G12 in KRAS and at Q61 in NRAS. These mutations are also known to be associated with patient prognosis (Non Patent Literature 3). Patent Literature 1 discloses a cyclic compound having selective KRAS inhibitory action on HRAS and NRAS.

Solid dispersions containing an insoluble compound and a polymeric compound (polymer) have been prepared to improve the solubility of a drug. For example, Patent Literature 2 and 3 describe that a solid dispersion containing a specific cyclic peptide compound and hydroxypropyl methylcellulose acetate succinate (HPMCAS) was prepared, and that a composition containing the solid dispersion and sodium lauryl sulfate (SLS) was prepared.

### Citation List

### Patent Literature

Patent Literature 1: International Publication No. WO 2023/214576
Patent Literature 2: International Publication No. WO 2023/063376
Patent Literature 3: International Publication No. WO 2024/080308

### Non Patent Literature

Non Patent Literature 1: Nat. Rev. Drug Discov., 2014, 13(11), pp. 828-851
Non Patent Literature 2: Nat. Rev. Drug Discov., 2014, 13(12), pp. 928-942
Non Patent Literature 3: Nat. Rev. Drug Discov., 2016, Vol. 15(11), pp. 771-785

### Summary of Invention

### Technical Problem

In the course of studying pharmaceutical formulations of a compound represented by formula (I), the present inventors have found that, in a formulation containing a solid dispersion of the compound represented by formula (I) and hydroxypropyl methylcellulose acetate succinate (HPMCAS), and sodium lauryl sulfate (SLS), it is possible to reduce the weight of the formulation required to achieve a certain level of solubility in simulated intestinal fluid (FaSSIF: Fasted State Simulated Intestinal Fluid) (hereinafter, the solubility in FaSSIF may be simply referred to as "solubility") by setting the content of HPMCAS in the solid dispersion to less than 1 part by mass per 1 part by mass of the compound represented by formula (I).

Patent Literature 2 and 3 neither disclose specific examples of a solid dispersion in which the content of a polymer is less than 1 part by mass per 1 part by mass of a specific cyclic peptide compound, nor disclose or suggest that the weight of a formulation required to achieve a certain level of solubility can be reduced by using such a composition.

An object of the present invention is to provide a solid dispersion capable of reducing the weight of a formulation required to achieve a certain level of solubility, and a composition containing the solid dispersion.

### Solution to Problem

The present invention encompasses each of the following inventions, for example:
[A-1] A solid dispersion comprising a component (1) that is one or more selected from the group consisting of a compound represented by formula (I), pharmaceutically acceptable salts thereof, and pharmaceutically acceptable solvates thereof, and a component (2) that is one or more polymers selected from the group consisting of hydroxypropyl methylcellulose and derivatives thereof, wherein a total content of the component (2) per 1 part by mass of a total content of the component (1) is more than 0.1 parts by mass and less than 1.0 part by mass. 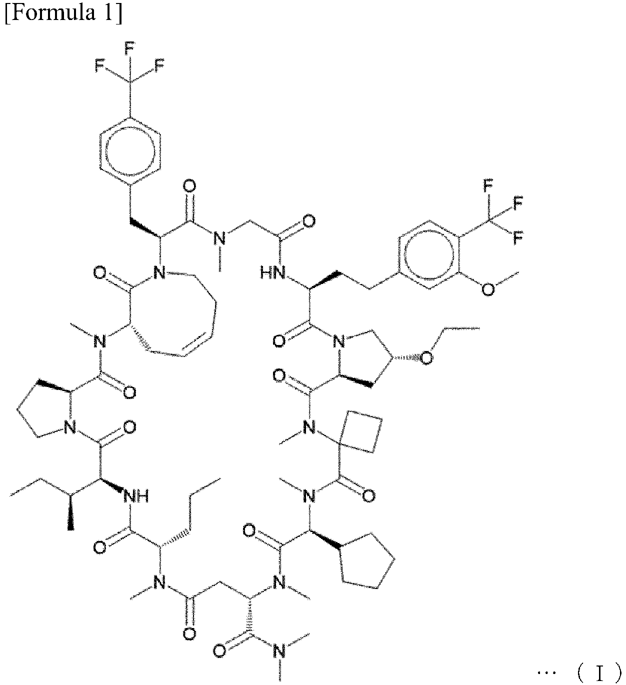
[A-2] The solid dispersion according to [A-1], wherein the component (1) is a compound represented by formula (I) or a solvate thereof.
[A-3] The solid dispersion according to [A-1] or [A-2], wherein the component (1) is a compound represented by formula (I) or a hydrate thereof.
[A-4] The solid dispersion according to any one of [A-1] to [A-3], wherein the component (1) is a compound represented by formula (I).
[A-5] The solid dispersion according to any one of [A-1] to [A-3], wherein the component (1) is a hydrate of the compound represented by formula (I).
[A-6] The solid dispersion according to any one of [A-1] to [A-5], wherein the component (2) is one or more polymers selected from the group consisting of hydroxypropyl methylcellulose and esters thereof.
[A-7] The solid dispersion according to any one of [A-1] to [A-6], wherein the component (2) is one or more polymers selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.
[A-8] The solid dispersion according to any one of [A-1] to [A-7], wherein the component (2) is one polymer selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.
[A-9] The solid dispersion according to any one of [A-1] to [A-8], wherein the component (2) is hydroxypropyl methyl cellulose acetate succinate (HPMCAS).
[A-10] The solid dispersion according to any one of [A-1] to [A-9], wherein a total content of the component (2) per 1 part by mass of a total content of the component (1) is 0.2 parts by mass or more and 0.7 parts by mass or less.
[A-11] The solid dispersion according to any one of [A-1] to [A-9], wherein a total content of the component (2) per 1 part by mass of a total content of the component (1) is 0.25 parts by mass or more and 0.6 parts by mass or less.
[A-12] The solid dispersion according to any one of [A-1] to [A-9], wherein a total content of the component (2) per 1 part by mass of a total content of the component (1) is 0.3 parts by mass or more and 0.5 parts by mass or less.
[A-13] The solid dispersion according to any one of [A-1] to [A-12], for use in combination with an alkyl sulfate.
[A-14] The solid dispersion according to [A-13], wherein an amount of the alkyl sulfate used in combination is 1.37 parts by mass or more and 6.66 parts by mass or less per 1 part by mass of a total content of the component (1).
[A-15] The solid dispersion according to [A-13], wherein an amount of the alkyl sulfate used in combination is 1.5 parts by mass or more and 5.0 parts by mass or less per 1 part by mass of a total content of the component (1).
[A-16] The solid dispersion according to [A-13], wherein an amount of the alkyl sulfate used in combination is 1.8 parts by mass or more and 4.0 parts by mass or less per 1 part by mass of a total content of the component (1).
[A-17] The solid dispersion according to [A-13], wherein an amount of the alkyl sulfate used in combination is 2.0 parts by mass or more and 3.0 parts by mass or less per 1 part by mass of a total content of the component (1).
[A-18] A composition comprising the solid dispersion according to any one of [A-1] to [A-17], and an alkyl sulfate.
[A-19] The composition according to [A-18], wherein a total content of the alkyl sulfate per 1 part by mass of a total content of the component (1) is 1.37 parts by mass or more and 6.66 parts by mass or less.
[A-20] The composition according to [A-18], wherein a total content of the alkyl sulfate per 1 part by mass of a total content of the component (1) is 1.5 parts by mass or more and 5.0 parts by mass or less per 1 part by mass of a total content of the component (1).
[A-21] The composition according to [A-18], wherein a total content of the alkyl sulfate per 1 part by mass of a total content of the component (1) is 1.8 parts by mass or more and 4.0 parts by mass or less per 1 part by mass of a total content of the component (1).
[A-22] The composition according to [A-18], wherein a total content of the alkyl sulfate per 1 part by mass of a total content of the component (1) is 2.0 parts by mass or more and 3.0 parts by mass or less per 1 part by mass of a total content of the component (1).
[A-23] The composition according to any one of [A-20] to [A-22], which is a composition for promoting absorption of the component (1).
[A-24] The composition according to any one of [A-20] to [A-23], which is a pharmaceutical composition.
[A-25] The composition according to [A-24], which is a pharmaceutical composition for administration.
[A-26] The composition according to [A-25], which is a pharmaceutical composition for oral administration.
[A-27] The composition according to any one of [A-18] to [A-26], in the form of a tablet.
[A-28] The composition according to any one of [A-24] to [A-27], wherein the component (1) is an active ingredient.
[A-29] The composition according to any one of [A-24] to [A-27], which is a pharmaceutical composition for treating or preventing cancer.
[A-30] The composition according to any one of [A-18] to [A-29], wherein the alkyl sulfate is a lauryl sulfate.
[A-31] The composition according to any one of [A-18] to [A-30], wherein the alkyl sulfate is sodium lauryl sulfate.
[B-1] A method for producing a solid dispersion, comprising a step of removing a solvent from a mixture comprising a component (1) that is one or more selected from the group consisting of a compound represented by formula (I), pharmaceutically acceptable salts thereof, and pharmaceutically acceptable solvates thereof, a component (2) that is one or more polymers selected from the group consisting of hydroxypropyl methylcellulose and derivatives thereof, and a solvent, wherein a total content of the component (2) per 1 part by mass of a total content of the component (1) is more than 0.1 parts by mass and less than 1.0 part by mass. 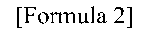 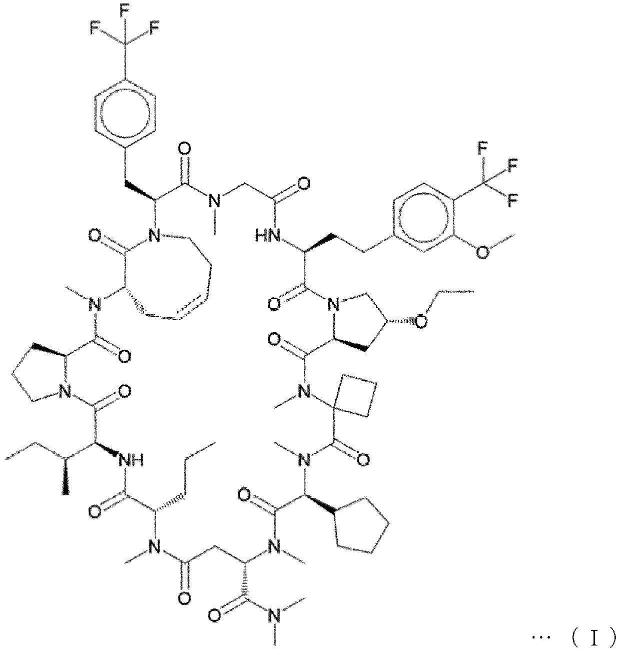
[B-2] The production method according to [B-1], wherein the solvent is removed by a spray-drying method.
[B-3] The production method according to [B-1] or [B-2], wherein the component (1) is a compound represented by formula (I) or a solvate thereof.
[B-4] The production method according to any one of [B-1] to [B-3], wherein the component (1) is a compound represented by formula (I) or a hydrate thereof.
[B-5] The production method according to any one of [B-1] to [B-4], wherein the component (1) is a compound represented by formula (I).
[B-6] The production method according to any one of [B-1] to [B-5], wherein the component (1) is a hydrate of the compound represented by formula (I).
[B-7] The production method according to [B-1] or [B-2], wherein the component (1) is a salt of the compound represented by formula (I).
[B-8] The production method according to any one of [B-1] to [B-7], wherein the component (2) is one or more polymers selected from the group consisting of hydroxypropyl methylcellulose and esters thereof.
[B-9] The production method according to any one of [B-1] to [B-8], wherein the component (2) is one or more polymers selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.
[B-10] The production method according to any one of [B-1] to [B-9], wherein the component (2) is one polymer selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.
[B-11] The production method according to any one of [B-1] to [B-10], wherein the component (2) is hydroxypropyl methyl cellulose acetate succinate (HPMCAS).
[B-12] The production method according to any one of [B-1] to [B-11], wherein a total content of the component (2) per 1 part by mass of a total content of the component (1) in the mixture is 0.2 parts by mass or more and 0.7 parts by mass or less.
[B-13] The production method according to any one of [B-1] to [B-11], wherein a total content of the component (2) per 1 part by mass of a total content of the component (1) in the mixture is 0.25 parts by mass or more and 0.6 parts by mass or less.
[B-14] The production method according to any one of [B-1] to [B-11], wherein a total content of the component (2) per 1 part by mass of a total content of the component (1) in the mixture is 0.3 parts by mass or more and 0.5 parts by mass or less.
[B-15] A method for producing a composition, comprising a step of mixing (mixing step) a solid dispersion obtained by the production method according to any one of [B-1] to [B-14] and an alkyl sulfate.
[B-16] The production method according to [B-15], wherein, in the mixing step, the alkyl sulfate is mixed in an amount of 1.37 parts by mass or more and 6.66 parts by mass or less per 1 part by mass of a total content of the component (1).
[B-17] The production method according to [B-15], wherein, in the mixing step, the alkyl sulfate is mixed in an amount of 1.5 parts by mass or more and 5.0 parts by mass or less per 1 part by mass of a total content of the component (1).
[B-18] The production method according to [B-15], wherein, in the mixing step, the alkyl sulfate is mixed in an amount of 1.8 parts by mass or more and 4.0 parts by mass or less per 1 part by mass of a total content of the component (1).
[B-19] The production method according to [B-15], wherein, in the mixing step, the alkyl sulfate is mixed in an amount of 2.0 parts by mass or more and 3.0 parts by mass or less per 1 part by mass of a total content of the component (1).
[B-20] The production method according to any one of [B-16] to [B-19], wherein the alkyl sulfate is a lauryl sulfate.
[B-21] The production method according to any one of [B-16] to [B-20], wherein the alkyl sulfate is sodium lauryl sulfate.

### Advantageous Effects of Invention

According to the present invention, a solid dispersion capable of reducing the weight of a formulation required to achieve a certain level of solubility, and a composition containing the solid dispersion, can be provided.

### Brief Description of Drawings

[Figure 1] Figure 1 shows the results of powder X-ray diffraction measurement of the solid dispersion obtained in Reference Example 1. The ordinate represents a diffraction intensity, and the abscissa represents a diffraction angle 2θ (°).
[Figure 2] Figure 2 shows the results of powder X-ray diffraction measurement of the solid dispersion obtained in Comparative Example 1a. The ordinate represents a diffraction intensity, and the abscissa represents a diffraction angle 2θ (°).
[Figure 3] Figure 3 shows the results of powder X-ray diffraction measurement of the solid dispersion obtained in Comparative Example 2. The ordinate represents a diffraction intensity, and the abscissa represents a diffraction angle 2θ (°).
[Figure 4] Figure 4 shows the results of powder X-ray diffraction measurement of the solid dispersion obtained in Example 1. The ordinate represents a diffraction intensity, and the abscissa represents a diffraction angle 2θ (°).
[Figure 5] Figure 5 shows the results of powder X-ray diffraction measurement of the solid dispersion obtained in Example 1b. The ordinate represents a diffraction intensity, and the abscissa represents a diffraction angle 2θ (°).
[Figure 6] Figure 6 shows the results of powder X-ray diffraction measurement of the solid dispersion obtained in Example 1-1. The ordinate represents a diffraction intensity, and the abscissa represents a diffraction angle 2θ (°).
[Figure 7] Figure 7 shows the results of powder X-ray diffraction measurement of the solid dispersion obtained in Example 2. The ordinate represents a diffraction intensity, and the abscissa represents a diffraction angle 2θ (°).
[Figure 8] Figure 8 shows the results of powder X-ray diffraction measurement of the solid dispersion obtained in Comparative Example 3. The ordinate represents a diffraction intensity, and the abscissa represents a diffraction angle 2θ (°).
[Figure 9] Figure 9 shows the results of powder X-ray diffraction measurement of the solid dispersion obtained in Comparative Example 4. The ordinate represents a diffraction intensity, and the abscissa represents a diffraction angle 2θ (°).
[Figure 10] Figure 10 shows the results of powder X-ray diffraction measurement of the solid dispersion obtained in Comparative Example 5. The ordinate represents a diffraction intensity, and the abscissa represents a diffraction angle 2θ (°).
[Figure 11] Figure 11 shows the results of powder X-ray diffraction measurement of the solid dispersion obtained in Comparative Example 6. The ordinate represents a diffraction intensity, and the abscissa represents a diffraction angle 2θ (°).
[Figure 12] Figure 12 shows the results of powder X-ray diffraction measurement of the solid dispersion obtained in Comparative Example 1b. The ordinate represents a diffraction intensity, and the abscissa represents a diffraction angle 2θ (°).
[Figure 13] Figure 13 shows the results of a dissolution test of measurement formulations 3-1 and 3-2 in Measurement Example 3. The ordinate represents the dissolved concentration of Peptide 1, and the abscissa represents the shaking time.
[Figure 14] Figure 14 shows the results of a dissolution test of each evaluation formulation in Evaluation Example 1. The ordinate represents the dissolved concentration of Peptide 1, and the abscissa represents the shaking time.
[Figure 15] Figure 15 shows the results of a dissolution test of each evaluation formulation in Evaluation Example 1. The ordinate represents the dissolved concentration of Peptide 1, and the abscissa represents the shaking time.
[Figure 16] Figure 16 shows the results of a dissolution test of evaluation formulation 1 in Evaluation Example 2-3. The ordinate represents the dissolved concentration of Peptide 1, and the abscissa represents the shaking time.
[Figure 17] Figure 17 shows the results of a dissolution test of evaluation formulation 3 in Evaluation Example 2-3. The ordinate represents the dissolved concentration of Peptide 1, and the abscissa represents the shaking time.
[Figure 18] Figure 18 shows the results of a dissolution test of evaluation formulation 5 in Evaluation Example 2-3. The ordinate represents the dissolved concentration of Peptide 1, and the abscissa represents the shaking time.

### Description of Embodiments

Hereinafter, the embodiments for carrying out the present invention are described in detail. However, the present invention is not limited by the embodiments given below.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, the term "to" that indicates a range includes values of both ends thereof. For example, "A to B" means the range of A or more and B or less.

The term "and/or" as used herein is meant to include every combination of the terms "and" and "or" appropriately combined. Specifically, for example, the term "A, B and/or C" includes the following seven variations; (i) A, (ii) B, (iii) C, (iv) A and B, (v) A and C, (vi) B and C, and (vii) A, B and C.

The solid dispersion according to the present embodiment comprises (1) one or more components selected from the group consisting of a compound represented by formula (I), pharmaceutically acceptable salts thereof, and pharmaceutically acceptable solvates thereof (also referred to herein as "component (1)"), and (2) one or more polymers selected from the group consisting of hydroxypropyl methylcellulose and derivatives thereof (also referred to herein as "component (2)"), wherein a total content of the component (2) per 1 part by mass of a total content of the component (1) is more than 0.1 parts by mass and less than 1.0 part by mass.

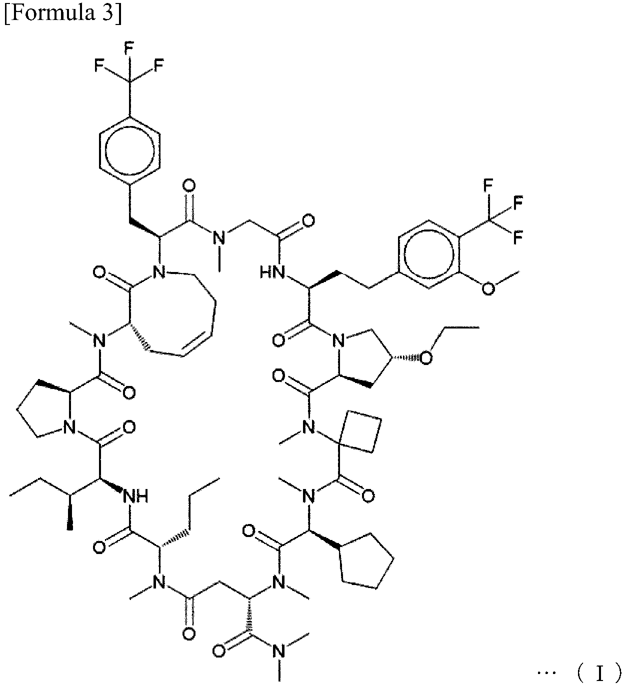

The compound represented by formula (I), pharmaceutically acceptable salts thereof, and pharmaceutically acceptable solvates thereof can be produced, for example, by the method disclosed in Patent Literature 1. In the present specification, the compound represented by formula (I) is also referred to as "Peptide 1." The IUPAC name of Peptide 1 is (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,8}.0^{26,30}]pentatetracont-42-ene-23,1'-cyclobutane]-17-carboxamide.

The salt of the compound represented by formula (I) may be any pharmaceutically acceptable salt, and specific examples thereof include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salts and potassium salts; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound represented by formula (I) into contact with an acid or a base which can be used for production of a medicament.

In the present specification, the solvate refers to a molecular aggregate formed by the compound represented by formula (I) or a pharmaceutically acceptable salt thereof together with a solvent. A solvate in which the solvent is water is also referred to as a hydrate. Solvates include not only solvates with a single solvent but also solvates with a plurality of solvents. Examples of solvates of the compound represented by formula (I) or a pharmaceutically acceptable salt thereof include solvates with solvents such as water, alcohol (e.g. methanol, ethanol, 1-propanol, and 2-propanol), and dimethylformamide. The solvate of the compound represented by formula (I) or a pharmaceutically acceptable salt thereof is preferably a hydrate, and specific examples of the hydrate include mono- to decahydrate, preferably mono- to pentahydrate, further preferably mono- to trihydrate.

The solid dispersion according to the present embodiment may contain, as the component (1), a compound represented by formula (I), a pharmaceutically acceptable salt thereof, or a pharmaceutically acceptable solvate thereof alone, or may contain two or more thereof.

The hydroxypropyl methyl cellulose is also known as hypromellose. As used herein, the term "derivative of hydroxypropyl methyl cellulose" means a polymer in which the hydroxyl group of hydroxypropyl methyl cellulose is reacted (modified), and examples thereof include an ester, ether, carbamate and carbonate of hydroxypropyl methyl cellulose. In the derivative of hydroxypropyl methyl cellulose according to the present embodiment, the proportion of modified hydroxyl groups in hydroxyl groups of hydroxypropyl methyl cellulose is not particularly limited, and may be, for example, 10% or more, 20% or more, 30% or more, 40% or more, 50% or more, 60% or more, 70% or more, or 80% or more.

The hydroxypropyl methyl cellulose or a derivative thereof according to the present embodiment is not particularly limited, and is preferably hydroxypropyl methyl cellulose or an ester thereof, more preferably hydroxypropyl methyl cellulose acetate succinate (HPMCAS), hydroxypropyl methyl cellulose phthalate (HPMCP), hydroxypropyl methyl acetate maleate or hydroxypropyl methyl trimellitate, and most preferably hydroxypropyl methyl cellulose acetate succinate (HPMCAS).

The solid dispersion according to the present embodiment may contain, as the component (2), hydroxypropyl methylcellulose or a derivative thereof alone, or may contain two or more thereof.

In the solid dispersion according to the present embodiment, a total content of the component (2) per 1 part by mass of a total content of the component (1) is more than 0.1 parts by mass and less than 1.0 part by mass. The total content of the component (2) per 1 part by mass of the total content of the component (1) may be, for example, 0.15 parts by mass or more, 0.2 parts by mass or more, 0.25 parts by mass or more, or 0.3 parts by mass or more, and may be 0.95 parts by mass or less, 0.9 parts by mass or less, 0.85 parts by mass or less, 0.8 parts by mass or less, 0.75 parts by mass or less, 0.7 parts by mass or less, 0.65 parts by mass or less, 0.6 parts by mass or less, 0.55 parts by mass or less, or 0.5 parts by mass or less. The range of the total content of the component (2) per 1 part by mass of the total content of the component (1) may be, for example, more than 0.1 parts by mass and less than 1.0 part by mass, preferably 0.2 parts by mass or more and 0.7 parts by mass or less, more preferably 0.25 parts by mass or more and 0.6 parts by mass or less, and most preferably 0.3 parts by mass or more and 0.5 parts by mass or less.

The content of the component (1) in the solid dispersion according to the present embodiment may be, for example, more than 50% by mass, 55% by mass or more, 60% by mass or more, 62.5% by mass or more, 65% by mass or more, 70% by mass or more, or 75% by mass or more based on the total amount of the solid dispersion. The content of the component (1) in the solid dispersion according to the present embodiment may be, for example, less than 100% by mass, 99% by mass or less, 97.5% by mass or less, 95% by mass or less, 92.5% by mass or less, 90% by mass or less, 87.5% by mass or less, 85% by mass or less, 82.5% by mass or less, or 80% by mass or less based on the total amount of the solid dispersion. The range of the content of the component (1) in the solid dispersion according to the present embodiment may be, for example, more than 50% by mass and less than 100% by mass, may be 60% by mass or more and 99% by mass or less, preferably 65% by mass or more and 95% by mass or less, more preferably 70% by mass or more and 90% by mass or less, and most preferably 75% by mass or more and 85% by mass or less based on the total amount of the solid dispersion. The content of the component (1) in the solid dispersion can be measured, for example, by a method using high-performance liquid chromatography (HPLC) as described in Examples below.

The content of the component (2) in the solid dispersion according to the present embodiment may be, for example, less than 50% by mass, 45% by mass or less, 40% by mass or less, 38.5% by mass or less, 35% by mass or less, 30% by mass or less, or 25% by mass or less based on the total amount of the solid dispersion. The content of the component (2) in the solid dispersion according to the present embodiment may be, for example, more than 0% by mass, 1% by mass or more, 2.5% by mass or more, 5% by mass or more, 7.5% by mass or more, 10% by mass or more, 12.5% by mass or more, 15% by mass or more, 17.5% by mass or more, or 20% by mass or more based on the total amount of the solid dispersion. The range of the content of the component (2) in the solid dispersion according to the present embodiment may be, for example, more than 0% by mass and less than 50% by mass, may be 1% by mass or more and 40% by mass or less, preferably 5% by mass or more and 35% by mass or less, more preferably 10% by mass or more and 30% by mass or less, and most preferably 15% by mass or more and 25% by mass or less based on the total amount of the solid dispersion. The content of the component (2) in the solid dispersion can be determined, for example, by subtracting the content (% by mass) of the substance to be dispersed contained in the solid dispersion measured by a method using high-performance liquid chromatography (HPLC) from 100% by mass.

The solid dispersion according to the present embodiment refers to a semi-solid or solid substance in which the component (1) and the component (2) are both present in a dispersed state. From the viewpoint of improving solubility, the component (1) and the component (2) are typically dispersed with uniformity, preferably dispersed with uniformity in very small units, and most preferably dispersed with uniformity at a molecular level, in a solid dispersion.

The method for forming a solid dispersion is not particularly limited, and examples thereof include a method in which a certain amount of the solvent or all the solvent is removed from a solution of the component (1) and the component (2) to form the solid dispersion, or a method in which a certain amount of a solvent is added to and simultaneously mixed with the component (1) and the component (2) that are solid. Specific examples thereof include a spray-drying method, a freeze-drying method, a precipitation method, a melt-extruding method, and a mixing/crushing method. A spray-drying method or a freeze-drying method is preferred, a spray-drying method or a freeze-drying method is more preferred, and a spray-drying method is most preferred. By using a spray-drying method, the component (1) and the component (2) in the solid dispersion can be brought into a more uniformly dispersed state, and solubility of a substance contained in the solid dispersion (for example, the component (1)) can be further improved. The solvent is only required to be capable of dissolving the component (1) and the component (2), and examples thereof include water, alcohol (for example, methanol, ethanol, n-propanol, iso-propanol and butanol), ketone (for example, acetone, methyl ethyl ketone and methyl iso-butyl ketone), an ester (for example, ethyl acetate and propyl acetate), acetonitrile, methylene chloride, toluene, 1,1,1-trichloroethane, and tetrahydrofuran. The solvent may be used alone, or used as a mixed solvent obtained by mixing two or more.

Solid dispersions are usually in the form of small particles. The volume average diameter of the particles may be less than 500 µm, less than 100 µm in diameter, less than 50 µm in diameter, or less than 25 µm in diameter. When the solid dispersion is formed by spray drying, the resulting dispersion takes such a form of small particles.

The solid dispersion according to the present embodiment can be obtained, for example, by a production method comprising a step of removing the solvent (removal step) from a mixture comprising a component (1) that is one or more selected from the group consisting of a compound represented by formula (I), pharmaceutically acceptable salts thereof, and pharmaceutically acceptable solvates thereof, a component (2) that is one or more polymers selected from the group consisting of hydroxypropyl methylcellulose and derivatives thereof, and a solvent, wherein a total content of the component (2) per 1 part by mass of a total content of the component (1) is more than 0.1 parts by mass and less than 1.0 part by mass.

The production method according to the present embodiment may comprise, in addition to the removal step, a step of preparing a mixture (preparation step) comprising a component (1) that is one or more selected from the group consisting of a compound represented by formula (I), pharmaceutically acceptable salts thereof, and pharmaceutically acceptable solvates thereof, a component (2) that is one or more polymers selected from the group consisting of hydroxypropyl methylcellulose and derivatives thereof, and a solvent, wherein a total content of the component (2) per 1 part by mass of a total content of the component (1) is more than 0.1 parts by mass and less than 1.0 part by mass.

The solvent used in the production method according to the present embodiment is not particularly limited as long as it is capable of dissolving the component (1) and the component (2), and examples thereof include water, alcohol (for example, methanol, ethanol, n-propanol, iso-propanol and butanol), ketone (for example, acetone, methyl ethyl ketone and methyl iso-butyl ketone), an ester (for example, ethyl acetate and propyl acetate), acetonitrile, methylene chloride, toluene, 1,1,1-trichloroethane, and tetrahydrofuran. The solvent may be used alone, or used as a mixed solvent obtained by mixing two or more.

The total content of the component (2) per 1 part by mass of the total content of the component (1) in the mixture may be, for example, 0.15 parts by mass or more, 0.2 parts by mass or more, 0.25 parts by mass or more, or 0.3 parts by mass or more, and may be 0.95 parts by mass or less, 0.9 parts by mass or less, 0.85 parts by mass or less, 0.8 parts by mass or less, 0.75 parts by mass or less, 0.7 parts by mass or less, 0.65 parts by mass or less, 0.6 parts by mass or less, 0.55 parts by mass or less, or 0.5 parts by mass or less. The range of the total content of the component (2) per 1 part by mass of the total content of the component (1) in the mixture may be, for example, more than 0.1 parts by mass and less than 1.0 part by mass, preferably 0.2 parts by mass or more and 0.7 parts by mass or less, more preferably 0.25 parts by mass or more and 0.6 parts by mass or less, and most preferably 0.3 parts by mass or more and 0.5 parts by mass or less.

In the production method according to the present embodiment, specific aspects of the component (1) and the component (2) are the same as those described above.

Examples of the method for removing the solvent in the removal step include a spray-drying method, a freeze-drying method, a precipitation method, a melt-extruding method, and a mixing/crushing method. A spray-drying method or a freeze-drying method is preferred, a spray-drying method or a freeze-drying method is more preferred, and a spray-drying method is most preferred.

The conditions for removing the solvent in the removal step can be appropriately set according to the types of the component (1), the component (2), and the solvent, the type of method for removing the solvent, and the like.

The composition according to the present embodiment contains the solid dispersion as described above and an alkyl sulfate.

The alkyl sulfate may be, for example, an alkyl sulfate having 6 to 18 carbon atoms (C6-C18; hereinafter, "Cp-Cq" means that the number of carbon atoms is p to q), preferably a C8-C16 alkyl sulfate, more preferably a C10-C14 alkyl sulfate, and most preferably a C12 alkyl sulfate (lauryl sulfate). The alkyl sulfate may be any pharmaceutically acceptable salt, and examples thereof include alkali metal salts such as sodium salts and potassium salts; and alkaline earth metal salts such as magnesium salts and calcium salts. These salts are produced by, for example, bringing an alkyl sulfuric acid into contact (neutralizing) with a base which can be used for production of a medicament. The alkyl sulfate is more preferably a lauryl sulfate, and most preferably sodium lauryl sulfate.

The composition according to the present embodiment may contain one alkyl sulfate alone, or may contain two or more thereof.

In the composition according to the present embodiment, a total content of the alkyl sulfate per 1 part by mass of a total content of the component (1) is 1.37 parts by mass or more and 6.66 parts by mass or less. The total content of the alkyl sulfate per 1 part by mass of the total content of the component (1) may be, for example, 1.37 parts by mass or more, 1.4 parts by mass or more, 1.5 parts by mass or more, 1.6 parts by mass or more, 1.7 parts by mass or more, 1.8 parts by mass or more, 1.9 parts by mass or more, or 2.0 parts by mass or more, and may be 6.66 parts by mass or less, 6.5 parts by mass or less, 6.0 parts by mass or less, 5.5 parts by mass or less, 5.0 parts by mass or less, 4.5 parts by mass or less, 4.0 parts by mass or less, 3.5 parts by mass or less, or 3.0 parts by mass or less. The range of the total content of the alkyl sulfate per 1 part by mass of the total content of the component (1) may be, for example, 1.37 parts by mass or more and 6.66 parts by mass or less, preferably 1.5 parts by mass or more and 5.0 parts by mass or less, more preferably 1.8 parts by mass or more and 4.0 parts by mass or less, and most preferably 2.0 parts by mass or more and 3.0 parts by mass or less.

The content of the solid dispersion contained in the composition according to the present embodiment may be, for example, more than 0% by mass and 90% by mass or less based on the total amount of the composition, and may be more than 0% by mass and 80% by mass or less, more than 0% by mass and 70% by mass or less, more than 0% by mass and 60% by mass or less, more than 0% by mass and 50% by mass or less, more than 0% by mass and 40% by mass or less, or more than 0% by mass and 30% by mass or less.

The composition according to the present embodiment can be obtained by a production method including a step of mixing (mixing step) the solid dispersion described above and an alkyl sulfate. The production method may include, in addition to the mixing step, a step of producing the solid dispersion described above (production step). The production step may be, for example, the one described in the method for producing the solid dispersion according to the present embodiment.

In the mixing step, the alkyl sulfate may be mixed in an amount of 1.37 parts by mass or more and 6.66 parts by mass or less per 1 part by mass of a total content of the component (1). In the mixing step, the mixing amount of the alkyl sulfate per 1 part by mass of the total content of the component (1) may be, as the total amount of the alkyl sulfate, for example, 1.37 parts by mass or more, 1.4 parts by mass or more, 1.5 parts by mass or more, 1.6 parts by mass or more, 1.7 parts by mass or more, 1.8 parts by mass or more, 1.9 parts by mass or more, or 2.0 parts by mass or more, and may be 6.66 parts by mass or less, 6.5 parts by mass or less, 6.0 parts by mass or less, 5.5 parts by mass or less, 5.0 parts by mass or less, 4.5 parts by mass or less, 4.0 parts by mass or less, 3.5 parts by mass or less, or 3.0 parts by mass or less. In the mixing step, the range of the mixing amount of the alkyl sulfate per 1 part by mass of the total content of the component (1) may be, as the total amount of the alkyl sulfate, for example, 1.37 parts by mass or more and 6.66 parts by mass or less, preferably 1.5 parts by mass or more and 5.0 parts by mass or less, more preferably 1.8 parts by mass or more and 4.0 parts by mass or less, and most preferably 2.0 parts by mass or more and 3.0 parts by mass or less.

In the production method according to the present embodiment, specific aspects of the alkyl sulfate are the same as those described above.

In the mixing step, in addition to the solid dispersion described above and the alkyl sulfate, other components may be mixed together therewith. The other components can be appropriately set according to the intended use of the resulting composition and the like. Specific examples of the other components include the pharmaceutically acceptable other components described below.

When the composition according to the present embodiment is a pharmaceutical composition, the pharmaceutical composition may contain, in addition to the solid dispersion described above and the alkyl sulfate, pharmaceutically acceptable other components to the extent that the effect according to the present invention is not impaired. The other components are not particularly limited as long as they are pharmaceutically acceptable, and examples include an excipient, a disintegrant, a glidant or lubricant, a taste and odor masking agent, a stabilizer, a pH adjusting agent, a preservative, and an antioxidant.

Examples of the excipient include lactose, corn starch, white sugar, glucose, mannitol, sorbit, starch, crystalline cellulose, silicon dioxide, and magnesium aluminate metasilicate. Examples of the disintegrant include sodium starch, pregelatinized starch, agar, gelatin powder, crystalline cellulose, calcium carbonate, sodium chloride, sodium bicarbonate, calcium citrate, anhydrous silicic acid, dextrin, pectin, carmellose, carmellose calcium, croscarmellose sodium, low-substituted hydroxypropyl cellulose, and sodium starch glycolate. Examples of the glidant or lubricant include light anhydrous silicic acid, aqueous silicic acid dioxide, magnesium stearate, and talc. Examples of the taste and odor masking agent include cocoa powder, menthol, aromatic powder, peppermint oil, borneol, and cinnamon powder. Examples of the stabilizer include phosphatidic acid, ascorbic acid, glycerin, and cetanol. Examples of the pH adjusting agent include lactic acid, succinic acid, gluconic acid, citric acid, citric acid hydrate, trisodium citrate, phosphoric acid, potassium carbonate, sodium hydrogen carbonate, tartaric acid, malic acid, ascorbic acid, fumaric acid, aspartic acid, glutamic acid, glutamic acid hydrochloride, malonic acid, maleic acid, meglumine, arginine, lysine, glycine, sodium carbonate, and sodium hydrogen phosphate. Examples of the preservative include ethyl paraoxybenzoate and propyl paraoxybenzoate. Examples of the antioxidant include butylated hydroxytoluene, butylated hydroxyanisole, propyl gallate, and gallic acid propyl ester.

When the composition according to the present embodiment is a pharmaceutical composition, the form of the pharmaceutical composition is not particularly limited, and is typically a solid. The pharmaceutical composition according to the present embodiments is molded into, for example, a powder, a subtle granule, a granule, a tablet, a coated tablet, a capsule, and the like, and used.

When the composition according to the present embodiment is a pharmaceutical composition, the pharmaceutical composition may be orally administered, or may be parenterally administered. Since the solubility of the component (1) (active ingredient) is enhanced, the pharmaceutical composition is preferably orally administered.

When the composition according to the present embodiment is a pharmaceutical composition, the subject to which the pharmaceutical composition is administered is not particularly limited, and may be a human or a non-human animal. Examples of the non-human animal include a dog, a monkey, a mini-pig, a rabbit, a rat, and a mouse.

When the composition according to the present embodiment is a pharmaceutical composition, the dosage of the pharmaceutical composition is not particularly limited, and it may be administered such that the dose of the component (1) (active ingredient) to be administered per body weight (kg) of the subject is 0.1 mg/kg or more and 1000 mg/kg or less, for example. The dosage of the component (1) (active ingredient) may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

The composition according to the present embodiments can be produced by being molded into an arbitrary dosage form by a method that is commonly used.

Since the solubility of the component (1) is enhanced in the composition (pharmaceutical composition) according to the present embodiment, it can be used as a composition for promoting absorption of the component (1).

Since the composition (pharmaceutical composition) according to the present embodiment contains the component (1) having selective KRAS inhibitory action on HRAS and NRAS, it can be used as a pharmaceutical composition for the treatment or prevention of cancer.

### Examples

Hereinafter, preferred specific aspects of the present invention are described by way of Examples, but the present invention is not limited thereto.

### [Synthesis Example 1: Method for Synthesizing Peptide 1]

Peptide 1 represented by the following formula (I) and the following name was synthesized by the same method as described in International Publication No. 2022/234853, and the final product was obtained as a dried product. Specifically, compound PP2320 in International Publication No. 2022/234853 corresponds to Peptide 1. 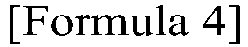 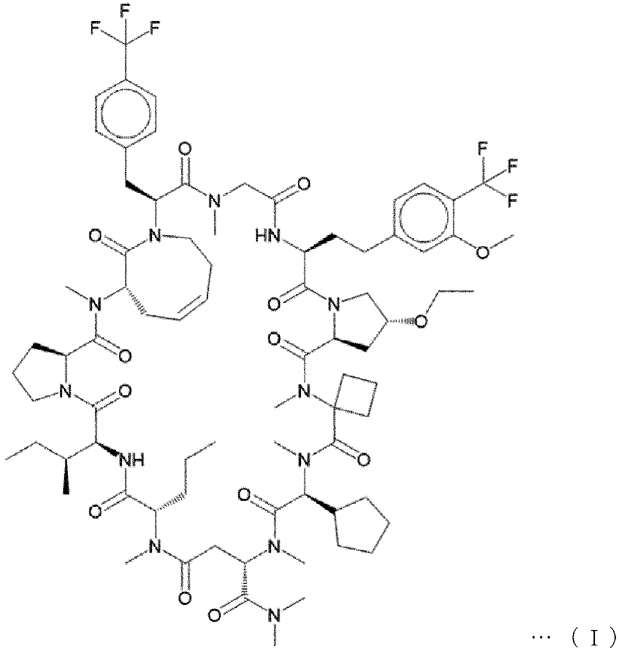 (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,1}.0^{26,30}]pentatetracont-42-ene-23,1'-cyclobutane]-17-carboxamide

### [Experimental Example 1: Preparation of Solid Dispersion Containing Peptide and Surfactant, and Measurement of Dissolved Concentration]

Peptide 1 and each surfactant listed in Table 1 were each dissolved in dimethyl sulfoxide (DMSO) in a predetermined amount to prepare a Peptide 1 solution and respective surfactant solutions. Subsequently, 50 µL each of the obtained Peptide 1 solution and each surfactant solution were mixed to obtain respective mixtures such that the concentration of Peptide 1 was 0.25 mg/mL and the Peptide 1/surfactant ratio was 1/2 (w/w). Each of the mixtures was freeze-dried, and then 50 µL of FaSSIF solution was added thereto, followed by shaking at 37°C and 1000 rpm for 10 minutes or 240 minutes. Each of the obtained suspensions was filtered and diluted with DMSO containing 4-hydroxyquinoline as an internal standard (IS). The FaSSIF solution was prepared using 3F Powder (FFF02) and FaSSIF buffer concentrate (FASBUF) obtained from Biorelevant, dissolved in a phosphate buffer containing sodium and chloride so that the concentrations of taurocholic acid and lecithin were 3 mmol/L and 0.75 mmol/L, respectively, and the pH was adjusted to 6.5 before use (hereinafter, the FaSSIF solution was prepared in the same manner in all experimental procedures). The dissolved concentration of Peptide 1 in FaSSIF solution in the presence of each surfactant was then quantified by HPLC/UV under the conditions described in Tables 2 and 3. The obtained results are shown in Table 4.

**[Table 1]**

| Generic Name | Trade Name | Abbreviation | Manufacturer |
|---|---|---|---|
| Sodium lauryl sulfate | SLS Fine | SLS | BASF |
| Sucrose fatty acid ester | DKester SS | DK ester | DKS Co. Ltd. |
| Polyoxyethylene hydrogenated castor oil 60 | HCO-60 | HCO-60 | Nippon Surfactant Industries/Nikko Chemicals |
| D-α-tocopherol polyethylene glycol 1000 succinate | D-α-tocopherol polyethylene glycol 1000 succinate | VitaminE TPGS | Merck |

**[Table 2]**

| ACQUITY UPLC Analysis Conditions | |
|---|---|
| Mobile phase | A: 0.05% TFA in H₂O |
| | B: 0.05% TFA in MeCN |
| Washing Solvent | Strong wash: 100% MeOH |
| | Weak wash: 10% MeCN |
| Purge Solvent | Same as A |
| Column | ACQUITY UPLC BEH Shield RP18 (50 mm × 2.1 mm × 1.7 µm) |
| Instrument Method | 5_98%B_84sec_S20C_C40C |
| Column Temperature | 40°C |
| Detection Method | PDA (IS: Chl 315 nm) |
| Injection Volume | 3 µL |
| Autosampler Temperature | 20°C |
| Analysis Time | 1.4 min |
| Flow Rate | 1.0 mL/min |

**[Table 3]**

| Liquid Delivery of Mobile Phase | | | |
|---|---|---|---|
| Time After Injection (min) | Mobile phase A (vol%) | Mobile phase B (vol%) | Flow Rate (mL/min) |
| 0.00 | 95 | 5 | 1.0 |
| 0.95 | 2 | 98 | 1.0 |
| 1.40 | 2 | 98 | 1.0 |
| 1.41 | 95 | 5 | 1.0 |

**[Table 4]**

| Surfactant | | DK ester | SLS | HCO-60 | VitaminE TPGS |
|---|---|---|---|---|---|
| Peptide 1 | 10 min | 35.8 | 110.4 | 33.2 | 67.8 |
| Dissolution Concentration (µg/mL) | 240 min | 21.0 | 79.6 | 20.6 | 51.8 |

### [Experimental Example 2: Preparation of Solid Dispersion Containing Peptide and Polymer, and Measurement of Dissolved Concentration]

Peptide 1 and each polymer listed in Table 5 were each dissolved in DMSO in a predetermined amount to prepare a Peptide 1 solution and respective polymer solutions. Subsequently, 50 µL each of the obtained Peptide 1 solution and each polymer solution were mixed to obtain respective mixed solutions such that the concentration of Peptide 1 was 0.25 mg/mL and the Peptide 1/polymer ratio was 1/2 (w/w). Each of the mixed solutions was freeze-dried, and then 50 µL of FaSSIF solution was added thereto, followed by shaking at 37°C and 1000 rpm for 10 minutes or 240 minutes. Each of the obtained suspensions was filtered and diluted with DMSO containing 4-hydroxyquinoline as an internal standard (IS). The dissolved concentration of Peptide 1 in FaSSIF solution in the presence of each polymer was then quantified by HPLC/UV under the conditions described in Tables 2 and 3. The obtained results are shown in Table 6.

**[Table 5]**

| Generic Name | Trade Name | Abbreviation | Manufacturer |
|---|---|---|---|
| Methylcellulose | METOLOSE SM-4 | MCSM-4 | Shin-Etsu Chemical |
| Methylcellulose | METOLOSE SM-15 | MCSM-15 | Shin-Etsu Chemical |
| Methylcellulose | METOLOSE SM-100 | MCSM-100 | Shin-Etsu Chemical |
| Hydroxypropyl cellulose | HPC SSL | HPC SSL | Nippon Soda |
| Hydroxypropyl cellulose | HPC L | HPC L | Nippon Soda |
| Hydroxypropyl cellulose | HPC M | HPC M | Nippon Soda |
| Hydroxypropyl methyl cellulose | HPMC(TC5) E | HPMC(TC5) E | Shin-Etsu Chemical |
| Hydroxypropyl methyl cellulose | HPMC(TC5) S | HPMC(TC5) S | Shin-Etsu Chemical |
| Povidone | Kollidone 30 | PVP K30 | BASF |
| Povidone | Kollidone 90 F | PVP K90 | BASF |
| Copovidone | Kollidone VA64 | PVP VA64 | BASF |
| Polyvinyl alcohol | Parteck MXP | PVA | Merck |
| Hydroxypropyl methyl cellulose acetate succinate | Shin-Etsu AQOAT (HPMC) AS-L | HPMC AS-L | Shin-Etsu Chemical |
| Hydroxypropyl methyl cellulose acetate succinate | Shin-Etsu AQOAT (HPMC) AS-M | HPMC AS-M | Shin-Etsu Chemical |
| Hydroxypropyl methyl cellulose acetate succinate | Shin-Etsu AQOAT (HPMC) AS-H | HPMC AS-H | Shin-Etsu Chemical |
| Dried methacrylic acid copolymer L | Eudragit L100 | Eudragit L100 | Evonik |
| Methacrylic acid copolymer LD | Eudragit L100-55 | Eudragit L100-55 | Evonik |
| Hydroxypropyl methyl cellulose phthalate | HPMCP HP-50 | HPMCP HP-50 | Shin-Etsu Chemical |
| Hydroxypropyl methyl cellulose phthalate | HPMCP HP-55 | HPMCP HP-55 | Shin-Etsu Chemical |
| Hydroxypropyl methyl cellulose phthalate | HPMCP HP-55S | HPMCP HP-55S | Shin-Etsu Chemical |
| Polyvinyl acetate phthalate | Phthalavin | PVAP | Colorcon |
| Cellulose acetate phthalate | Cellulose acetate phthalate | CAP | Merck |
| Carboxymethyl ethyl cellulose | CMEC | CMEC | Sanyo Chemical/Freund Corporation |
| Polyethylene glycol | Macrogol 4000 | PEG4000 | Sanyo Chemical |

**[Table 6]**

| Polymer | Peptide 1 Dissolution Concentration (µg/mL) | |
|---|---|---|
| | 10 min | 240 min |
| No polymer | 1.1 | 0.5 |
| MCSM-4 | 5.4 | 2.4 |
| MCSM-15 | 7.2 | 4.4 |
| MCSM-100 | 7.1 | 2.5 |
| HPC SSL | 6.9 | 2.7 |
| HPC L | 5.9 | 1.6 |
| HPC M | 1.3 | 0.3 |
| HPMC(TC5) E | 4.4 | 3.9 |
| HPMC(TC5) S | 4.2 | 2.8 |
| PVP K30 | 3.8 | 1.5 |
| PVP K90 | 4.4 | 2.3 |
| PVP VA64 | 5.1 | 3.1 |
| PVA | 9.8 | 9.3 |
| HPMC AS-L | 5.0 | 4.4 |
| HPMC AS-M | 1.5 | 1.1 |
| HPMC AS-H | 1.2 | 2.4 |
| Eudragit L100 | 8.7 | 12.7 |
| Eudragit L100-55 | 3.0 | 4.2 |
| HPMCP HP-50 | 3.8 | 5.8 |
| HPMCP HP-55 | 4.2 | 4.9 |
| HPMCP HP-55S | 4.0 | 4.2 |
| PVAP | 4.7 | 6.0 |
| CAP | 9.0 | 9.4 |
| CMEC | 2.4 | 1.9 |
| PEG4000 | 1.4 | 0.6 |

### [Reference Example 1, Comparative Examples 1a, 1b and 2 to 6, and Examples 1, 1b, 1-1 and 2: Preparation of Solid Dispersion]

### (Reference Example 1)

Peptide 1 and Eudragit L100 were added to ethanol such that the weight ratio of Peptide 1 and Eudragit L100 was 1:2 and the solid concentration was 8 wt/vol%, thereby preparing a solution. The solution was spray-dried to obtain a solid dispersion.

### (Comparative Examples 1a and 1b)

Peptide 1 and HPMC AS-L were added to acetone such that the weight ratio of Peptide 1 and HPMC AS-L was 1:2 and the solid concentration was 12 wt/vol%, thereby preparing a suspension. The suspension was spray-dried to obtain a solid dispersion.

### (Comparative Example 2)

Peptide 1 and HPMC AS-L were added to acetone such that the weight ratio of Peptide 1 and HPMC AS-L was 1:1 and the solid concentration was 8 wt/vol%, thereby preparing a suspension. The suspension was spray-dried to obtain a solid dispersion.

### (Example 1)

Peptide 1 and HPMC AS-L were added to acetone such that the weight ratio of Peptide 1 and HPMC AS-L was 1:0.5 and the solid concentration was 6 wt/vol%, thereby preparing a suspension. The suspension was spray-dried to obtain a solid dispersion.

### (Example 1b)

Peptide 1 and HPMC AS-L were added to acetone such that the weight ratio of Peptide 1 and HPMC AS-L was 1:0.5 and the solid concentration was 6 wt/vol%, thereby preparing a suspension. The suspension was spray-dried to obtain a solid dispersion.

### (Example 1-1)

Peptide 1 and HPMC (TC5) E were added to a solution of methanol and water at a volume ratio of 9:1 such that the weight ratio of Peptide 1 and HPMC (TC5) E was 1:0.5 and the solid concentration was 6 wt/vol%, thereby preparing a suspension. The suspension was spray-dried to obtain a solid dispersion.

### (Example 2)

Peptide 1 and HPMC AS-L were added to acetone such that the weight ratio of Peptide 1 and HPMC AS-L was 1:0.3 and the solid concentration was 5.2 wt/vol%, thereby preparing a suspension. The suspension was spray-dried to obtain a solid dispersion.

### (Comparative Example 3)

Peptide 1 and HPMC AS-L were added to acetone such that the weight ratio of Peptide 1 and HPMC AS-L was 1:0.1 and the solid concentration was 4.4 wt/vol%, thereby preparing a suspension. The suspension was spray-dried to obtain a solid dispersion.

### (Comparative Example 4)

Peptide 1 was added to acetone such that the concentration was 4 wt/vol%, thereby preparing a solution. The solution was spray-dried to obtain a solid dispersion.

### (Comparative Example 5)

Peptide 1 and PVP VA64 were added to ethanol such that the weight ratio of Peptide 1 and PVP VA64 was 1:0.5 and the solid concentration was 6 wt/vol%, thereby preparing a solution. The solution was spray-dried to obtain a solid dispersion.

### (Comparative Example 6)

Peptide 1 and Eudragit L100 were added to ethanol such that the weight ratio of Peptide 1 and Eudragit L100 was 1:0.5 and the solid concentration was 6 wt/vol%, thereby preparing a solution. The solution was spray-dried to obtain a solid dispersion.

### [Measurement Example 1: Powder X-ray Diffraction Measurement of Solid Dispersion Containing Peptide and Polymer]

The solid dispersions obtained in Reference Example 1, Comparative Examples 1a, 1b and 2 to 6, and Examples 1, 1b, 1-1 and 2 were each subjected to powder X-ray diffraction measurement by the following measurement method.
Measurement apparatus: D8 Discover, 2D VANTEC-500 solid state detector (manufactured by Bruker)
Radiation source: CuKα
Tube voltage and tube current: 40 kV and 40 mA or 50 kV and 1,000 µA
Measurement range: 5 to 31°
Exposure time: 40 to 80 seconds

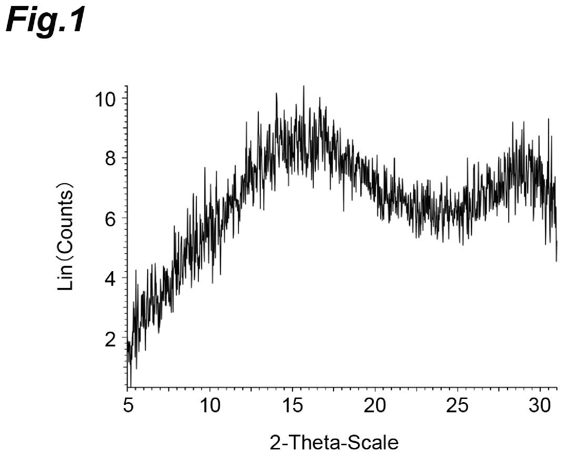

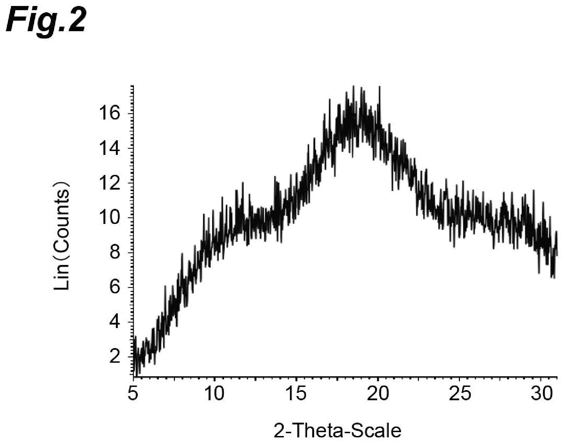

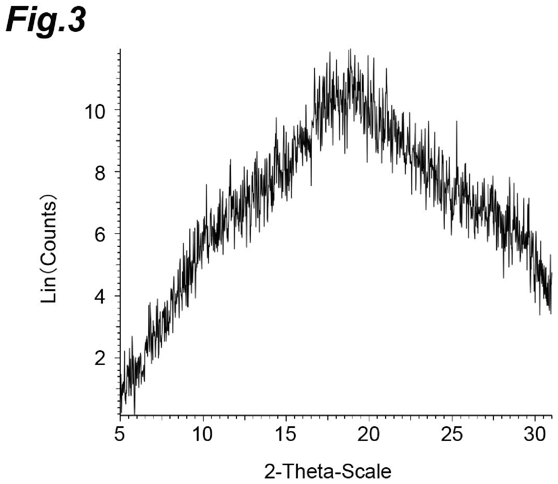

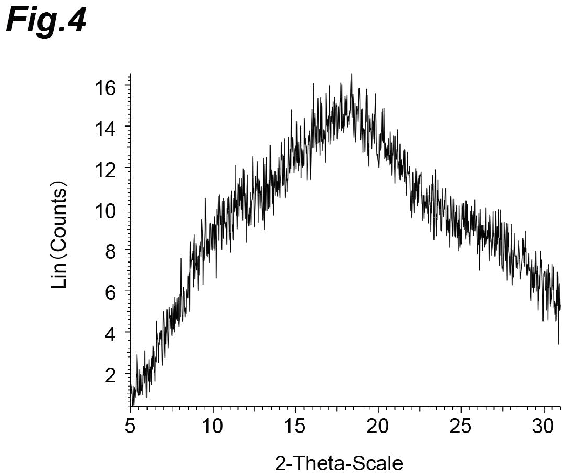

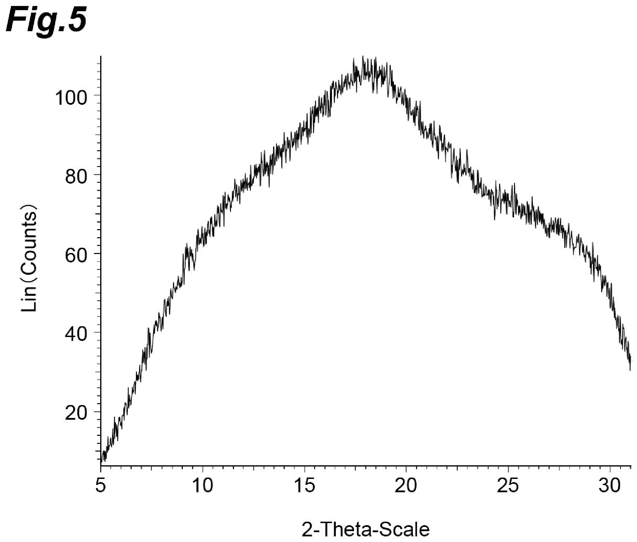

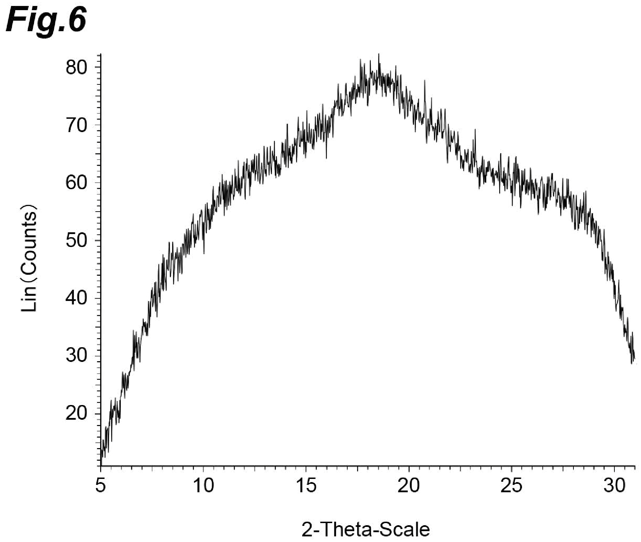

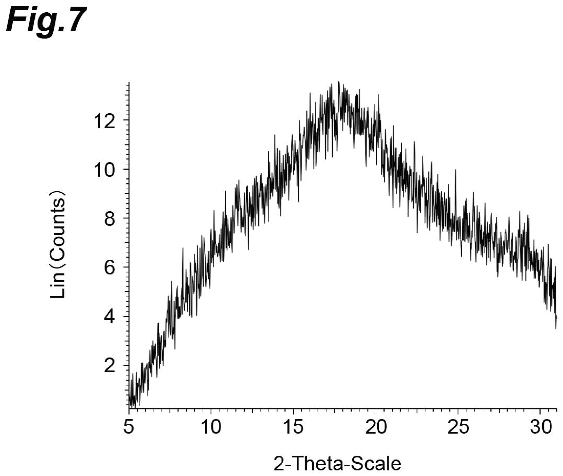

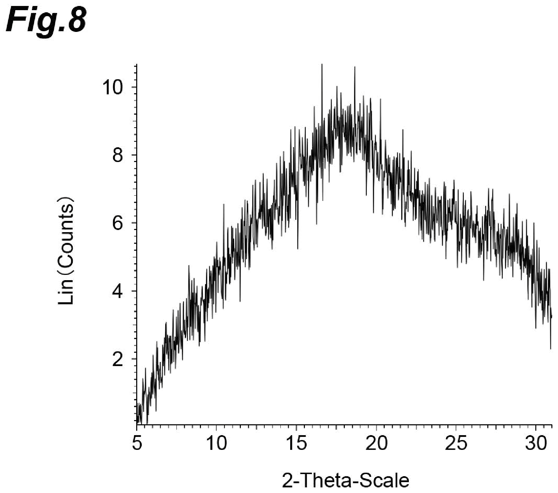

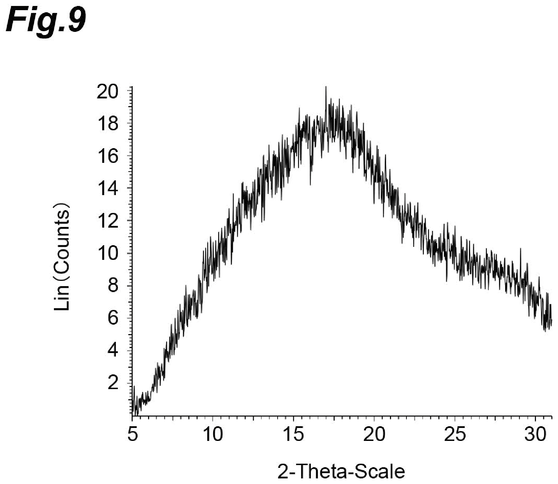

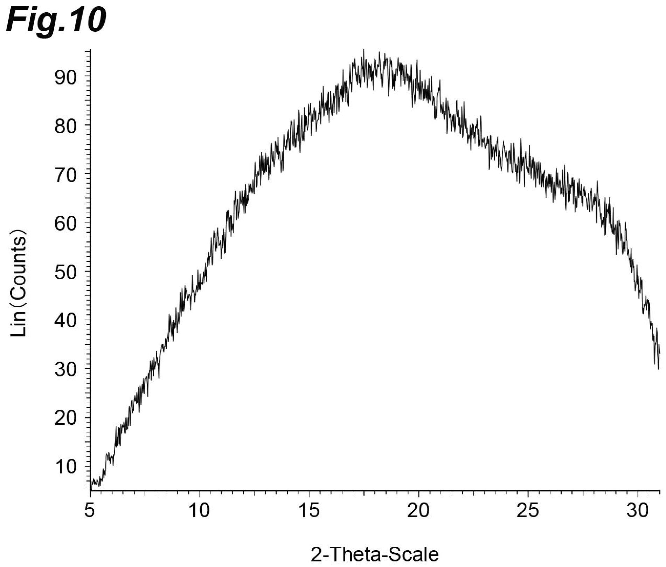

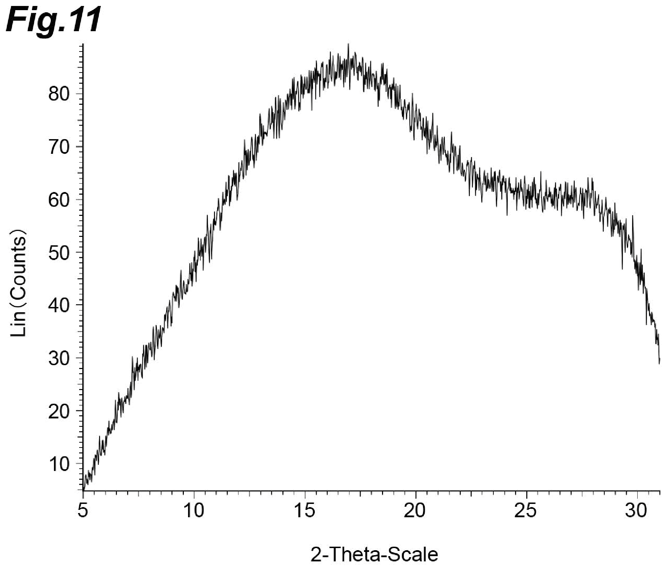

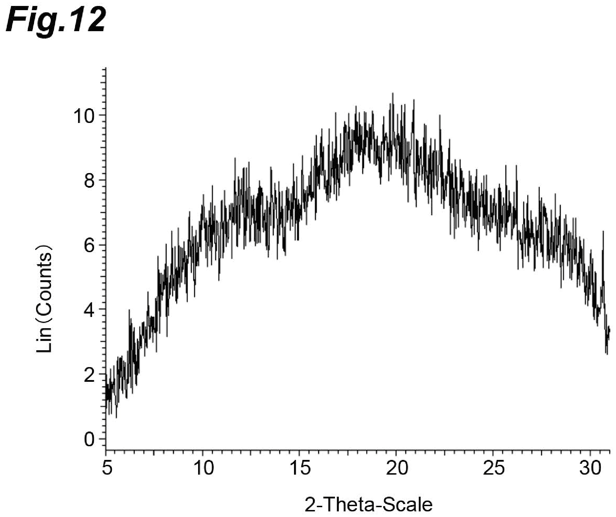

The results are shown in Figures 1 to 12. As a result of the measurement, a halo pattern was observed in all of the solid dispersions.

### [Measurement Example 2: Content of Peptide 1]

The content of Peptide 1 in the solid dispersions obtained in Reference Example 1, Comparative Examples 1a, 1b and 2 to 6, and Examples 1, 1b, 1-1 and 2 was measured under the following conditions.

### <Content Measurement: Solid Dispersions of Reference Example 1 and Comparative Example 1a>

The solid dispersions obtained in Reference Example 1 and Comparative Example 1a were weighed into glass vials in an amount of 1 mg as a formulation and dissolved in dimethyl acetamide. The sample was diluted with dimethyl acetamide containing 2-(1-naphthyl)ethanol as an internal standard and analyzed by LC/UV under the conditions described in Table 7. The results are shown in Table 10.

**[Table 7]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 um, 2.1@50 mm | A) 0.05% TFA/H₂O | 95/5(initial) => 2/98 | 1 | 40 | 0.7 | PDA | 281 (API), 282 (IS) |
| | | B) 0.05% TFA/MeCN | (0.95 min) => 2/98 | | | | | |
| | | | (0.45 min) => 95/5 | | | | | |
| | | | (0.01 min) | | | | | |

### <Content Measurement: Solid Dispersions of Comparative Examples 1b and 2 to 6, and Examples 1, 1b, 1-1 and 2>

The solid dispersions obtained in Comparative Examples 1b and 2 to 6, and Examples 1, 1b, 1-1 and 2 were weighed into glass vials in an amount of 1 mg as a formulation and diluted with dimethyl acetamide containing 2-(1-naphthyl)ethanol as an internal standard. Comparative Examples 1b and 2 to 4, and Examples 1 and 2 were analyzed by LC/UV under the conditions described in Table 8, and Comparative Examples 5 and 6, and Examples 1b and 1-1 were analyzed by LC/UV under the conditions described in Table 9. The results are shown in Table 10.

**[Table 8]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 um, 2.1@50 mm | A) 0.05% TFA/H₂O | 95/5(initial) => 2/98 | 1 | 60 | 0.5 | PDA | 220 (API), 220 (IS) |
| | | B) 0.05% TFA/MeCN | (0.95 min) => 2/98 | | | | | |
| | | | (0.45 min) => 95/5 | | | | | |
| | | | (0.01 min) | | | | | |

**[Table 9]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 um, 2.1@50 mm | A) 0.05% TFA/H₂O | 95/5(initial) => 2/98 | 1 | 40 | 1.0 | PDA | 282 (API), 281 (IS) |
| | | B) 0.05% TFA/MeCN | (0.95 min) => 2/98 | | | | | |
| | | | (0.45 min) => 95/5 | | | | | |
| | | | (0.01 min) | | | | | |

**[Table 10]**

| | Content of Peptide 1 (mass%) |
|---|---|
| Solid dispersion of Reference Example 1 | 32.3 |
| Solid dispersion of Comparative Example 1a | 32.7 |
| Solid dispersion of Comparative Example 1b | 31.9 |
| Solid dispersion of Comparative Example 2 | 50.0 |
| Solid dispersion of Example 1 | 67.3 |
| Solid dispersion of Example 1b | 65.7 |
| Solid dispersion of Example 1-1 | 67.3 |
| Solid dispersion of Example 2 | 76.3 |
| Solid dispersion of Comparative Example 3 | 90.4 |
| Solid dispersion of Comparative Example 4 | 99.4 |
| Solid dispersion of Comparative Example 5 | 68.6 |
| Solid dispersion of Comparative Example 6 | 66.5 |

### [Measurement Example 3: Dissolution Test]

The solid dispersion obtained in Reference Example 1 or Comparative Example 1a, lactose (Pharmatose 200M, manufactured by DFE Pharma), and SLS were weighed in the amounts described in Table 11 and mixed to prepare measurement formulations 3-1 and 3-2, which are lactose mixtures.

**[Table 11]**

| | Measurement formulation 3-1 | Measurement formulation 3-2 |
|---|---|---|
| Solid dispersion of Reference Example 1 (mg) | 38.2 | - |
| Solid dispersion of Comparative Example 1a (mg) | - | 38.7 |
| SLS(mg) | 25.0 | 25.0 |
| Pharmatose 200M(mg) | 11.8 | 11.3 |
| Total (mg) | 75.0 | 75.0 |
| Content of Peptide 1 in evaluation formulation(mg) | 12.5 | 12.5 |

For measurement formulations 3-1 and 3-2, the dissolved concentration was measured using a 708-DS dissolution apparatus from Agilent Technologies, Inc. The amount of the measurement formulation used for the measurement was 75 mg (containing 12.5 mg as Peptide 1), the conditions of the dissolution test were a rotation speed of 50 rpm, and 50 mL of FaSSIF solution was used as the test solution. Sampling was performed using an 850-DS sampling station from Agilent Technologies, Inc., and sample filtration was performed using a Whatman 850-DS 8-channel filter plate. The obtained samples were diluted with a dimethyl acetamide/ethylene glycol mixed solution containing 2-(1-naphthyl)ethanol as an internal standard and analyzed by LC/UV under the conditions described in Table 12.

**[Table 12]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 µm, 2.1@50 mm | A) 0.05% TFA/H₂O | 95/5(initial) => 2/98 | 1 | 40 | 5 | PDA | 225 (API), 282 (IS) |
| | | B) 0.05% TFA/MeCN | (0.95 min) => 2/98 | | | | | |
| | | | (0.45 min) => 95/5 | | | | | |
| | | | (0.01 min) | | | | | |

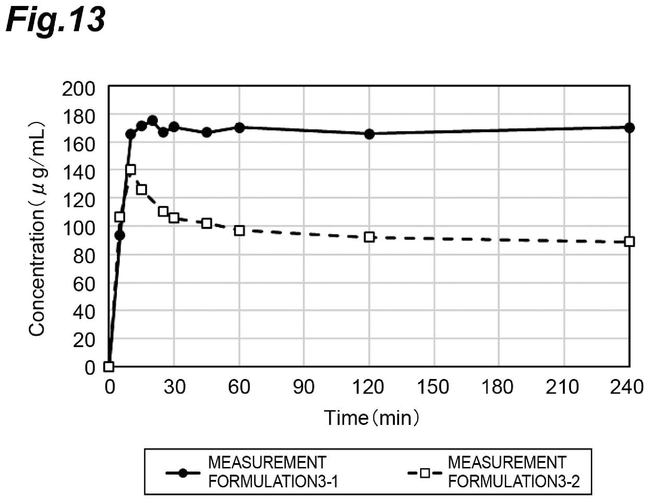

The results of the dissolution test are shown in Figure 13. From the results of the dissolution test, it was confirmed that measurement formulation 3-1 exhibited higher dissolution as compared with measurement formulation 3-2. From these results, it was confirmed that HPMC AS-L is suitable as a polymer for preparing a solid dispersion containing Peptide 1.

### [Evaluation Example 1: Dissolved Concentration and Formulation Weight]

The solid dispersions obtained in Comparative Examples 1b and 2 to 6, and Examples 1, 1b, 1-1 and 2, SLS, and lactose (Pharmatose 200M, manufactured by DFE Pharma) were weighed in the amounts described in Table 13 and mixed to prepare evaluation formulations 1 to 10.

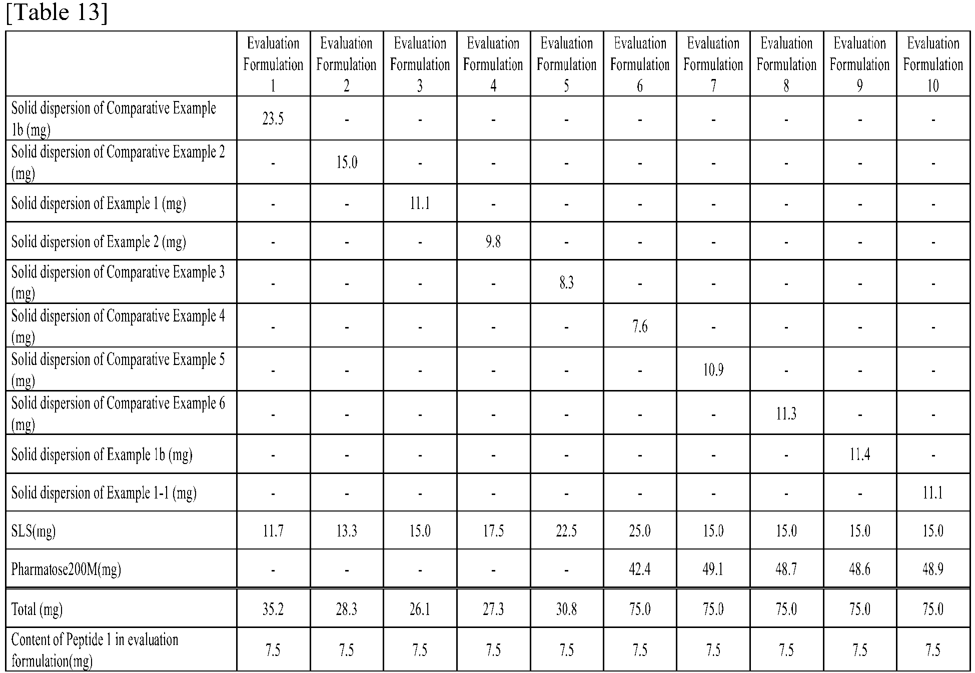

For evaluation formulations 1 to 10, the dissolution properties were evaluated using a 708-DS dissolution apparatus from Agilent Technologies, Inc. The amounts of the evaluation formulations were the amounts described in "Total (mg)" in Table 13 (each containing 7.5 mg as Peptide 1), the conditions of the dissolution test were a rotation speed of 50 rpm, and 50 mL of FaSSIF solution was used as the test solution. Sampling was performed using an 850-DS sampling station from Agilent Technologies, Inc., and sample filtration was performed using a Whatman 850-DS 8-channel filter plate. The obtained samples were diluted with a dimethyl acetamide/ethylene glycol mixed solution containing 2-(1-naphthyl)ethanol as an internal standard and analyzed by LC/UV under the conditions described in Table 14.

**[Table 14]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 µm, 2.1@50 mm | A) 0.05% TFA/H₂O | 95/5(initial) => 2/98 | 1 | 40 | 5 | PDA | 225 (API), 282 (IS) |
| | | B) 0.05% TFA/MeCN | (0.95 min) => 2/98 | | | | | |
| | | | (0.45 min) => 95/5 | | | | | |
| | | | (0.01 min) | | | | | |

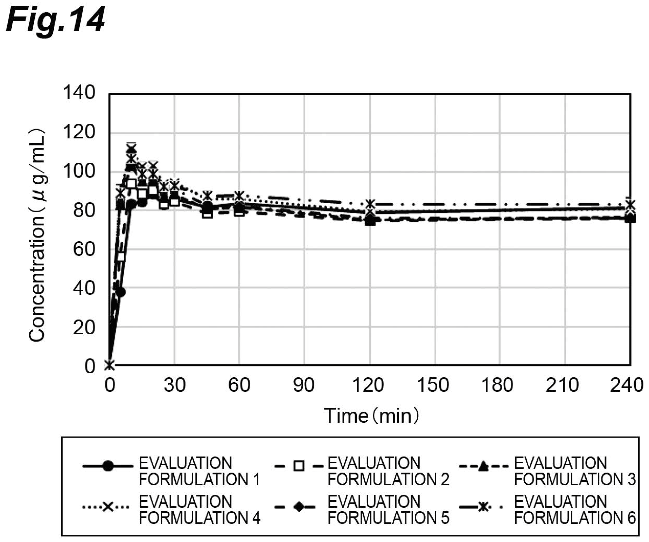

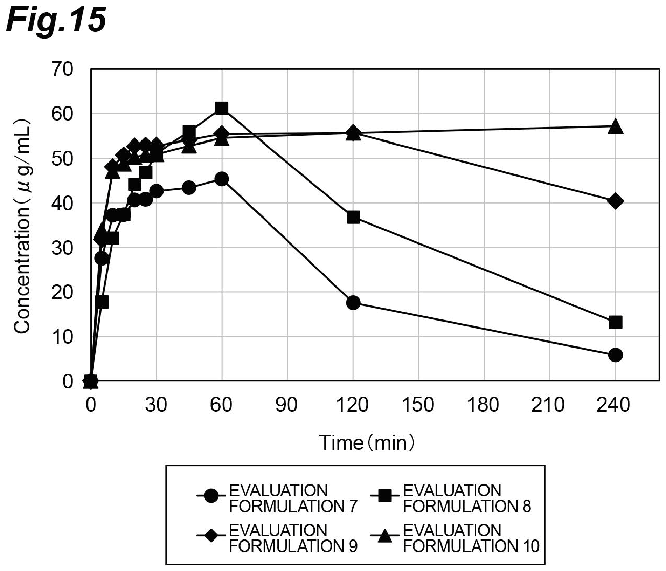

The results of the dissolution test are shown in Table 15 and Figures 14 and 15.

**[Table 15]**

| | Dissolved Concentration (µg/mL) | | | | | | | | | | |
|---|---|---|---|---|---|---|---|---|---|---|---|
| | 0 min | 5 min | 10 min | 15 min | 20 min | 25 min | 30 min | 45 min | 60 min | 120 min | 240 min |
| Evaluation formulation 1 | 0.0 | 37.7 | 83.1 | 84.5 | 88.3 | 83.0 | 85.8 | 81.7 | 83.4 | 79.0 | 81.3 |
| Evaluation formulation 2 | 0.0 | 56.2 | 93.7 | 88.7 | 90.5 | 83.3 | 84.6 | 78.6 | 79.5 | 74.7 | 76.2 |
| Evaluation formulation 3 | 0.0 | 83.0 | 103.1 | 95.1 | 96.0 | 86.5 | 88.3 | 80.8 | 81.5 | 75.0 | 76.7 |
| Evaluation formulation 4 | 0.0 | 88.0 | 111.9 | 102.7 | 103.0 | 93.8 | 94.2 | 85.7 | 85.9 | 79.4 | 81.0 |
| Evaluation formulation 5 | 0.0 | 86.9 | 110.6 | 100.7 | 100.6 | 92.2 | 91.0 | 82.8 | 82.1 | 75.8 | 76.1 |
| Evaluation formulation 6 | 0.0 | 88.9 | 107.0 | 99.0 | 98.7 | 92.4 | 92.7 | 87.5 | 87.7 | 83.3 | 83.0 |
| Evaluation formulation 7 | 0.0 | 27.5 | 37.2 | 37.3 | 40.6 | 40.8 | 42.6 | 43.4 | 45.3 | 17.5 | 5.8 |
| Evaluation formulation 8 | 0.0 | 17.7 | 32.0 | 37.4 | 44.1 | 46.8 | 51.1 | 55.9 | 61.1 | 36.8 | 13.2 |
| Evaluation formulation 9 | 0.0 | 31.8 | 48.0 | 50.6 | 52.6 | 52.7 | 52.8 | 54.0 | 55.4 | 55.6 | 40.4 |
| Evaluation formulation 10 | 0.0 | 33.8 | 47.1 | 48.7 | 50.2 | 50.7 | 50.9 | 52.8 | 54.5 | 55.6 | 57.1 |

The results of the dissolution test showed that evaluation formulations 1 to 6 were all formulations having equivalent dissolution properties at 240 minutes after the start of the dissolution test. In addition, in evaluation formulations 5, 4 and 3, the dissolved concentrations at 5 minutes and 10 minutes were higher than those of evaluation formulations 2 and 1. The results showed that a formulation having a Peptide 1:polymer ratio of 1:0.5 or less has a superior initial dissolution rate. In addition, the dissolved concentrations of evaluation formulations 9 and 10 at 120 minutes and 240 minutes were higher than the dissolved concentrations of evaluation formulations 7 and 8 at 120 minutes and 240 minutes. Furthermore, in evaluation formulations 3 and 4, the weight (mg) of the formulation was lower than that of the other evaluation formulations, and it was shown that they are formulations having good dissolution properties with a smaller amount of additives. Of these, evaluation formulation 3 had the lowest weight among evaluation formulations 1 to 6, and it was shown that it is a formulation having good dissolution properties with the smallest amount of additives. In addition, the solid dispersions obtained in Examples 1 and 2 are considered to have superior tablet disintegration properties because the weight ratio of HPMC AS-L to Peptide 1 is small, being less than 1. From these results, it was confirmed that hydroxypropyl methylcellulose or a derivative thereof is suitable as a polymer for preparing a solid dispersion containing Peptide 1.

### [Evaluation Example 2: Stability Evaluation]

### (Evaluation Example 2-1: Chemical Stability)

For evaluation formulations 11 and 12, each reagent was weighed as described in Table 16 so that the amount of Peptide 1 was 1 mg, placed in glass vials, and mixed. Subsequently, the mixtures were placed in aluminum pouches containing an oxygen-absorbing desiccant, sealed, and stored in a thermostatic chamber at 40°C for 6 months. To evaluate chemical stability, the samples before and after storage were dissolved in N,N-dimethyl acetamide containing hexyl benzoate as an internal standard, and the peak intensity of Peptide 1 (the peak intensity of Peptide 1 in a fixed amount of the evaluation formulation) was analyzed by LC/UV under the conditions described in Table 17.

**[Table 16]**

| | Evaluation formulation 11 | Evaluation formulation 12 |
|---|---|---|
| Solid dispersion of Comparative Example 1a (mg) | 3.06 | - |
| Peptide 1 (mg) | - | 1.00 |
| SLS(mg) | 3.00 | 3.00 |
| Total(mg) | 6.06 | 4.00 |
| Content of Peptide 1 in evaluation formulation(mg) | 1.00 | 1.00 |

**[Table 17]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Sigma-Aldrich BIOshell A160 peptide C18 Column, 2.7 µm, 2.1@150 mm | A) 0.05% TFA/H₂O | 50/50(initial) | 0.3 | 50 | 2 | PDA | 273 |
| | | | => 0/100 (15 min) | | | | | |
| | | B) 0.05% | | | | | | |
| | | TFA/MeCN | => 0/100 (5 min) | | | | | |
| | | | => 50/50 (0.1 min) | | | | | |

Then, the residual rate was determined by the following equation. 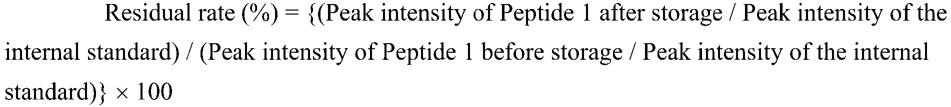 Residual rate (%) = {(Peak intensity of Peptide 1 after storage/Peak intensity of the internal standard)/(Peak intensity of Peptide 1 before storage/Peak intensity of the internal standard)} × 100

The results are shown in Table 18.

**[Table 18]**

| Evaluation Formulation | Storage Conditions | Residual Rate (%) |
|---|---|---|
| Evaluation formulation 11 | 40°C for 6 months (oxygen-absorbing desiccant) | ≥98 |
| Evaluation formulation 12 | 40°C for 6 months (oxygen-absorbing desiccant) | ≥98 |

As shown in Table 18, in all of the evaluation formulations, Peptide 1 exhibited a high residual rate of 98% or more under storage conditions at 40°C for 6 months in the presence of an oxygen-absorbing desiccant, and it was demonstrated that Peptide 1 is chemically stable. From these results, it can be understood that Peptide 1 is chemically stable at least within a range in which the weight ratio of Peptide 1 and HPMC AS-L is 1:2 to 1:0.

### (Evaluation Example 2-2: Physical Stability)

For evaluation formulations 11 and 12, one spatulaful of each was scooped into a glass vial. Subsequently, the samples were placed in a glass desiccator containing a sufficient amount of a saturated aqueous sodium chloride solution to maintain a constant humidity during the storage period, sealed, and stored in a thermostatic chamber at 40°C for 6 months. It is considered that, based on Journal of Research of the National Bureau of Standards - A. Physics and Chemistry, Vol. 81A, No. 1, January-February 1977, the humidity in the desiccator during the storage period was maintained at approximately 75% RH. To evaluate physical stability, Peptide 1 before and after storage was analyzed by the following measurement method.
Measurement apparatus: D8 Discover, 2D VANTEC-500 solid state detector (manufactured by Bruker)
Radiation source: CuKα
Tube voltage and tube current: 50 kV and 1000 µA
Measurement range: 5 to 31°
Exposure time: 120 seconds

The results are shown in Table 19.

**[Table 19]**

| Physical Stability, Powder X-ray Diffraction Measurement | | |
|---|---|---|
| Evaluation Formulation | Storage Conditions | Powder X-ray Diffraction |
| Evaluation formulation 11 | 40°C for 6 months (saturated aqueous sodium chloride solution) | Maintained amorphous state |
| Evaluation formulation 12 | 40°C for 6 months (saturated aqueous sodium chloride solution) | Maintained amorphous state |

As shown in Table 19, in all of the evaluation formulations, Peptide 1 maintained an amorphous state under storage conditions at 40°C for 6 months in the presence of a saturated aqueous sodium chloride solution, and it was demonstrated that Peptide 1 is physically stable. From these results, it can be understood that amorphous Peptide 1 is physically stable at least within a range in which the weight ratio of Peptide 1 and HPMC AS-L is 1:2 to 1:0.

### (Evaluation Example 2-3: Dissolved Concentration Stability)

For evaluation formulations 1, 3 and 5, 33.8 mg as Peptide 1 was weighed into a glass vial, respectively. Subsequently, the samples were placed in a glass desiccator containing a sufficient amount of a saturated aqueous sodium chloride solution to maintain a constant humidity during the storage period, sealed, and stored in a thermostatic chamber at 40°C for 1 or 3 months. It is considered that, based on Journal of Research of the National Bureau of Standards - A. Physics and Chemistry, Vol. 81A, No. 1, January-February 1977, the humidity in the desiccator during the storage period was maintained at approximately 75% RH. To evaluate physical stability, a subsequent Peptide 1 dissolution test was conducted using each evaluation formulation before and after storage. The results are shown in Table 20.

**[Table 20]**

| Evaluation Formulation | Storage Conditions | Peptide 1 Dissolution Test |
|---|---|---|
| Evaluation formulation 1 | 40°C for 3 months (saturated aqueous sodium chloride solution) | Dissolution maintained |
| Evaluation formulation 3 | 40°C for 3 months (saturated aqueous sodium chloride solution) | Dissolution maintained |
| Evaluation formulation 5 | 40°C for 1 month (saturated aqueous sodium chloride solution) | Dissolution maintained |

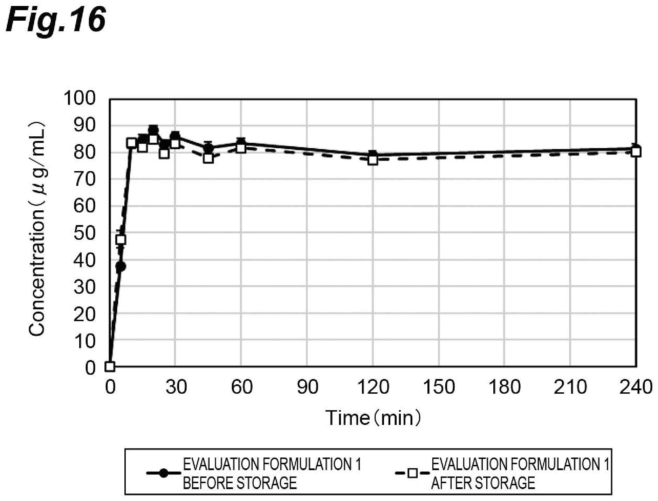

For evaluation formulations 1, 3, and 5, the dissolution properties were evaluated using a 708-DS dissolution apparatus from Agilent Technologies, Inc. The amounts of the evaluation formulations used for the evaluation were the amounts described in "Total (mg)" in Table 13 (each containing 7.5 mg as Peptide 1), the conditions of the dissolution test were a rotation speed of 50 rpm, and 50 mL of FaSSIF solution was used as the test solution. Sampling was performed using an 850-DS sampling station from Agilent Technologies, Inc., and sample filtration was performed using a Whatman 850-DS 8-channel filter plate. The obtained samples were diluted with a dimethyl acetamide/ethylene glycol mixed solution containing 2-(1-naphthyl)ethanol as an internal standard and analyzed by LC/UV under the conditions described in Table 21. The results are shown in Figures 16 to 18.

**[Table 21]**

| Instrument | Column | Mobile phase | Gradient (A/B) | Flow rate (mL/min) | Column Temperature (°C) | Injection Volume (µL) | Detection Method | Analytical Wavelength (nm) |
|---|---|---|---|---|---|---|---|---|
| Waters UPLC H-Class | Waters ACQUITY UPLC BEH Shield RP18, 1.7 um, 2.1@50 mm | A) 0.05% TFA/H₂O | 95/5(initial) => 2/98 | 1 | 40 | 5 | PDA | 225 (API), 282 (IS) |
| | | B) 0.05% TFA/MeCN | (0.95 min) => 2/98 | | | | | |
| | | | (0.45 min) => 95/5 | | | | | |
| | | | (0.01 min) | | | | | |

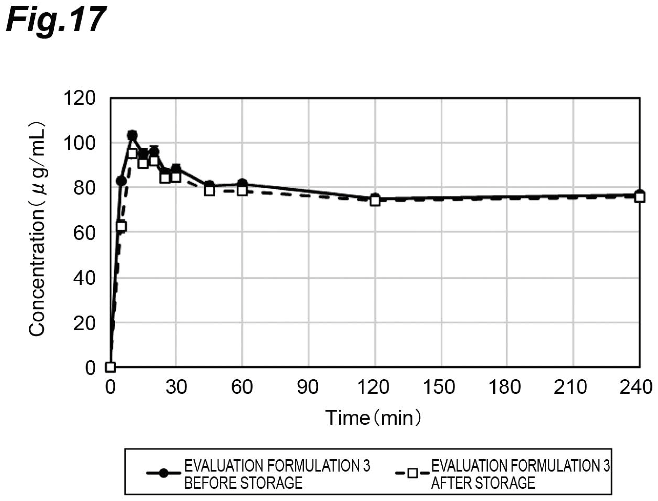

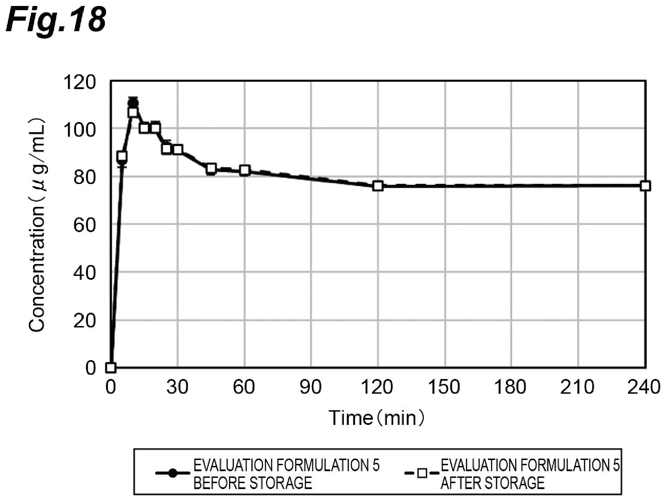

Figure 16 shows the results of the dissolution test of evaluation formulation 1. Figure 17 shows the results of the dissolution test of evaluation formulation 3. Figure 18 shows the results of the dissolution test of evaluation formulation 5. As shown in Table 20 and Figures 16 to 18, in all of the solid dispersion-containing formulations, the peptide maintained its dissolution properties under storage conditions at 40°C for 1 or 3 months in the presence of a saturated aqueous sodium chloride solution, and it was shown that the peptide is physically stable. From these results, it can be understood that amorphous Peptide 1 is physically stable at least within a range in which the weight ratio of Peptide 1 and HPMC AS-L is 1:2 to 1:0.

### (Evaluation Example 2-4: Monkey PK Test)

To evaluate the absorbability of Peptide 1 in the solid dispersion obtained in Example 1, evaluation formulation 13, in which SLS and lactose (Pharmatose 200M, manufactured by DFE Pharma) were mixed, was prepared with the composition described in Table 22. The evaluation formulation was administered to monkeys so that the dose of Peptide 1 was 3 mg/kg, and a PK test was conducted. As a result of the test, it was confirmed that the exposure of Peptide 1 was sufficiently high, with an AUC (0-48 h) of 4750 (ng·h/mL) and a Cmax of 510 (ng/mL).

**[Table 22]**

| | Evaluation formulation 13 |
|---|---|
| Solid dispersion of Example 1 (mg) | 111.3 |
| SLS(mg) | 149.8 |
| Pharmatose200M(mg) | 1113.9 |
| Total (mg) | 1375.0 |
| Content of Peptide 1 in evaluation formulation (mg) | 74.9 |

## Claims

1. A solid dispersion comprising a component (1) that is one or more selected from the group consisting of a compound represented by formula (I), pharmaceutically acceptable salts thereof, and pharmaceutically acceptable solvates thereof, and a component (2) that is one or more polymers selected from the group consisting of hydroxypropyl methylcellulose and derivatives thereof, wherein a total content of the component (2) per 1 part by mass of a total content of the component (1) is more than 0.1 parts by mass and less than 1.0 part by mass.

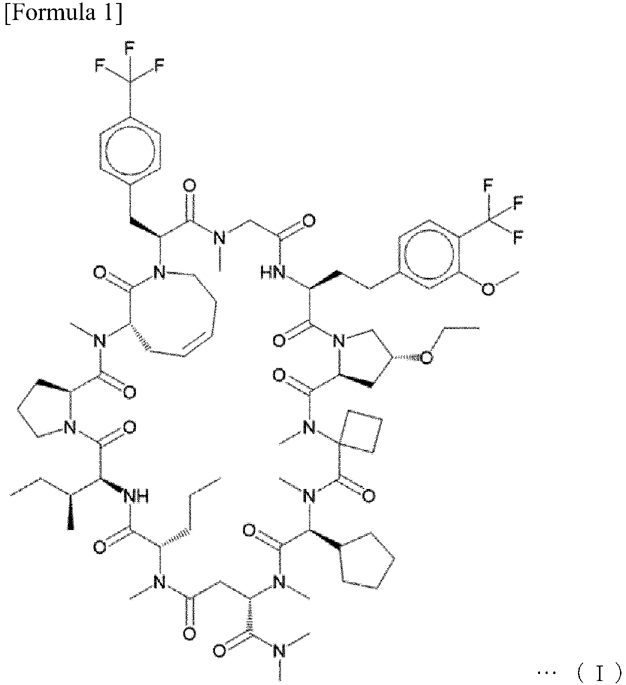

2. The solid dispersion according to claim 1, wherein the component (1) is a compound represented by formula (I).

3. The solid dispersion according to claim 1 or 2, wherein the component (2) is one or more polymers selected from the group consisting of hydroxypropyl methylcellulose and esters thereof.

4. The solid dispersion according to any one of claims 1 to 3, wherein the component (2) is one or more polymers selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.

5. The solid dispersion according to any one of claims 1 to 4, wherein the component (2) is one polymer selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.

6. The solid dispersion according to any one of claims 1 to 5, wherein the component (2) is hydroxypropyl methyl cellulose acetate succinate (HPMCAS).

7. A composition comprising the solid dispersion according to any one of claims 1 to 6, and an alkyl sulfate.

8. The composition according to claim 7, wherein a total content of the alkyl sulfate per 1 part by mass of a total content of the component (1) is 1.37 parts by mass or more and 6.66 parts by mass or less.

9. The composition according to claim 7 or 8, which is a pharmaceutical composition.

10. The composition according to claim 9, which is a pharmaceutical composition for oral administration.

11. The composition according to claim 9 or 10, in the form of a tablet.

12. The composition according to any one of claims 9 to 11, wherein the component (1) is an active ingredient.

13. The composition according to any one of claims 7 to 12, wherein the alkyl sulfate is a lauryl sulfate.

14. The composition according to any one of claims 7 to 13, wherein the alkyl sulfate is sodium lauryl sulfate.

15. A method for producing a solid dispersion, comprising a step of removing a solvent from a mixture comprising a component (1) that is one or more selected from the group consisting of a compound represented by formula (I), pharmaceutically acceptable salts thereof, and pharmaceutically acceptable solvates thereof, a component (2) that is one or more polymers selected from the group consisting of hydroxypropyl methylcellulose and derivatives thereof, and a solvent, wherein a total content of the component (2) per 1 part by mass of a total content of the component (1) is more than 0.1 parts by mass and less than 1.0 part by mass.
